(19) Europäisches Patentamt
European Patent Office
Office européen des brevets

(11) Publication number: **0 326 322**
**A1**

# (12) EUROPEAN PATENT APPLICATION

(21) Application number: **89300641.1**

(22) Date of filing: **24.01.89**

(51) Int. Cl.[4]: **A 61 K 47/00**
A 61 K 39/395

(30) Priority: **27.01.88 US 148822**

(43) Date of publication of application:
**02.08.89 Bulletin 89/31**

(84) Designated Contracting States:
**AT BE CH DE ES FR GB GR IT LI LU NL SE**

(71) Applicant: **ELI LILLY AND COMPANY**
**Lilly Corporate Center**
**Indianapolis Indiana 46285 (US)**

(72) Inventor: **Starling, James Jacob**
**1126 Hillcrest Drive**
**Carmel Indiana 46032 (US)**

**Laguzza, Bennett Coleman**
**7340 North Grand Avenue**
**Indianapolis Indiana 46250 (US)**

(74) Representative: **Hudson, Christopher Mark et al**
**Erl Wood Manor**
**Windlesham Surrey GU20 6PH (GB)**

Claims for the following Contracting States: ES + GR.

(54) **Antibody conjugates.**

(57) Immunoglobulin conjugates are formed by reaction of an antineoplastic indole-dihydroindole vinca alkaloid containing a hydrazide group attached at C-3 or C-4 with an oxidized IgG antibody having unusually high carbohydrate content, and are of the formula

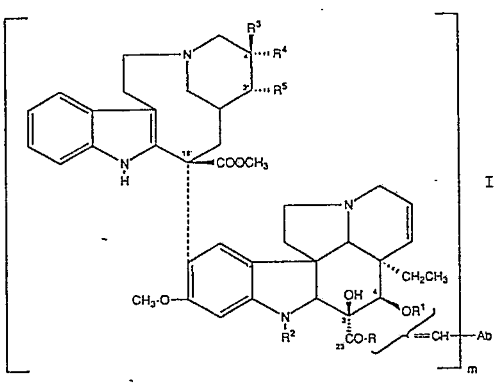

I

wherein $R^2$ is H, $CH_3$ or CHO; when $R^4$ and $R^5$ are taken singly, $R^5$ is H, and one of $R^3$ and $R^4$ is ethyl and the other is H or OH; when $R^4$ and $R^5$ are taken together with the carbons to which they are attached, they form an oxirane ring in which case $R^3$ is ethyl; R is NHN=,

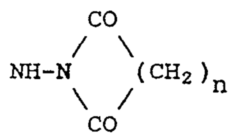

,

O($C_{1-3}$ alkyl), $NH_2$, NH($C_{1-3}$ alkyl), NH-$CH_2CH_2$-Y, 1-pyrrolidinyl or 1-piperidinyl, wherein n is 2-4 and Y is Cl, $OCH_3$ or $SCH_3$; $R^1$ is H, ($C_{1-3}$ alkyl)-CO, chloro-substituted ($C_{1-3}$ alkyl)-CO or $R^6$ wherein $R^6$ is COXCONHN= wherein X is $C_{1-4}$ straight chain alkylene, $C_{2-8}$ branched chain alkylene, $C_{2-4}$ alkenylene, $C_{3-4}$ alkenylene, $C_{3-6}$ cycloalkylene, phenylene, hydroxy-substituted $C_{1-4}$ alkylene, or a direct bond, provided that either R is NHN= or $R^1$ is $R^6$, but R cannot be NHN= when $R^1$ is $R^6$ and $R^1$ cannot be $R^6$ when R is NHN=; m is an integer from about 5 to about 35; Ab is an antibody of the IgG class, containing from about 6% to about 18% of carbohydrate.

EP 0 326 322 A1

## Description

### ANTIBODY CONJUGATES

The present invention belongs to the fields of immunology and pharmaceutical chemistry, and provide new conjugates of antibodies and vinca drugs.

Immunologists and chemists have recently combined to develop conjugates composed of an immunoglobulin which targets a defined antigen, and a drug or other active agent which is taken to the desired site by the immunoglobulin. Many such conjugates have been described in the literature. For example, British patent applications 2137202 and 2137210 describe conjugates of vinca alkaloids, which are anticancer drugs, with immunoglobulins which target tumor antigens.

Despite the scientific effort which has been devoted to such conjugates, the field may still fairly be said to be in its early development. Among the problems which are not yet well understood is the difficulty of loading a large amount of the active agent on the immunoglobulin without interfering with its ability to bind to the antigen. One solution to the problem is provided by the present invention, which permits synthesis of conjugates having high ratios of drug to immunoglobulin.

The present invention provides conjugates of the formula

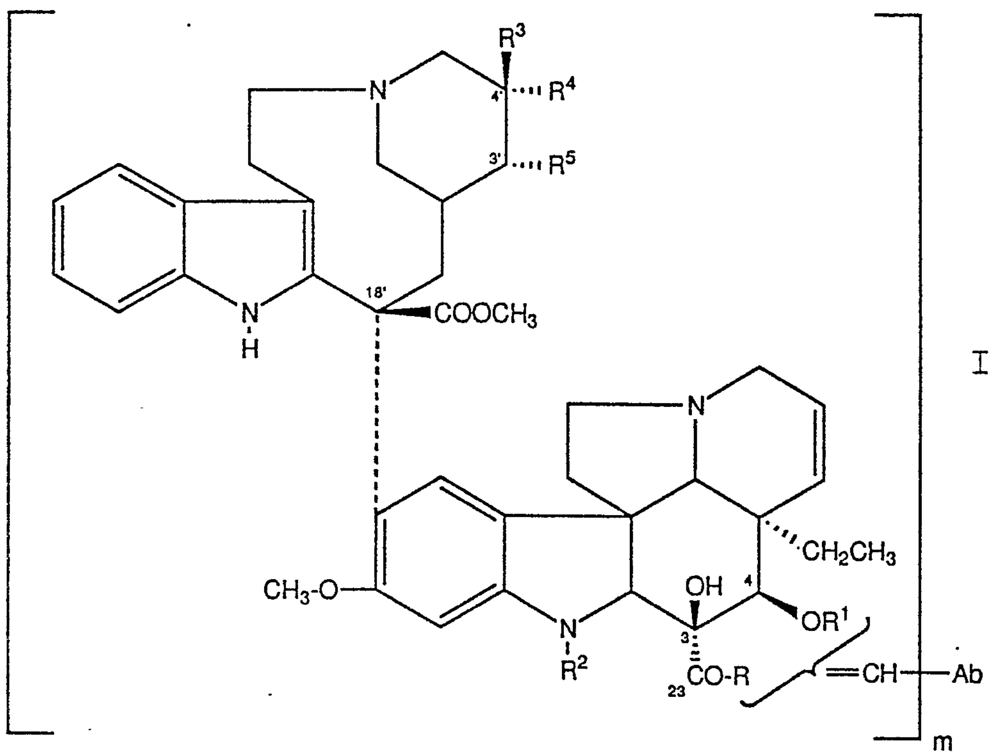

I

wherein $R^2$ is H, $CH_3$ or CHO; when $R^4$ and $R^5$ are taken singly, $R^5$ is H, and one of $R^3$ and $R^4$ is ethyl and the other is H or OH; when $R^4$ and $R^5$ are taken together with the carbons to which they are attached, they form an oxirane ring in which case $R^3$ is ethyl; R is NHN=,

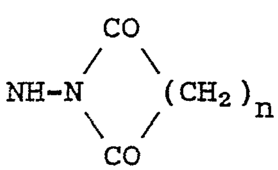

,

O($C_{1-3}$ alkyl), $NH_2$, NH($C_{1-3}$ alkyl), NH-$CH_2CH_2$-Y, 1-pyrrolidinyl or 1-piperidinyl, wherein n is 2-4 and Y is Cl, $OCH_3$ or $SCH_3$; $R^1$ is H, ($C_{1-3}$ alkyl)-CO, chloro-substituted ($C_{1-3}$ alkyl)-CO or $R^6$ wherein $R^6$ is COXCONHN= wherein X is $C_{1-4}$ straight chain alkylene, $C_{2-8}$ branched chain alkylene, $C_{2-4}$ alkenylene, $C_{3-4}$ alkynylene, $C_{3-6}$ cycloalkylene, phenylene, hydroxy-substituted $C_{1-4}$ alkylene, or a direct bond, provided that either R is NHN=

or $R^1$ is $R^6$, but R cannot be NHN= when $R^1$ is $R^6$ and $R^1$ cannot be $R^6$ when R is NHN=; m is an integer from about 5 to about 35; Ab is an antibody of the IgG class, containing from about 6% to about 18% of carbohydrate.

Groups illustrative of X in the above formula include methylene, ethylene, propylene, butylene, vinyl, propylene, butenylene, butynylene, propynylene, hydroxyethylene, 1,2-dihydroxyethylene, 1,2-dimethylethylene, 1,2,3,4-tetrahydroxybutylene, 3,4-dimethylbutylene, 1,4-cyclohexylene, 1,4-phenylene, 1,2-phenylene, and the like.

The invention also provides a process for preparing the conjugates of formula I by reacting an oxidized antibody of the IgG class containing from about 6% to about 18% of carbohydrate, with a vinca hydrazide of the formula

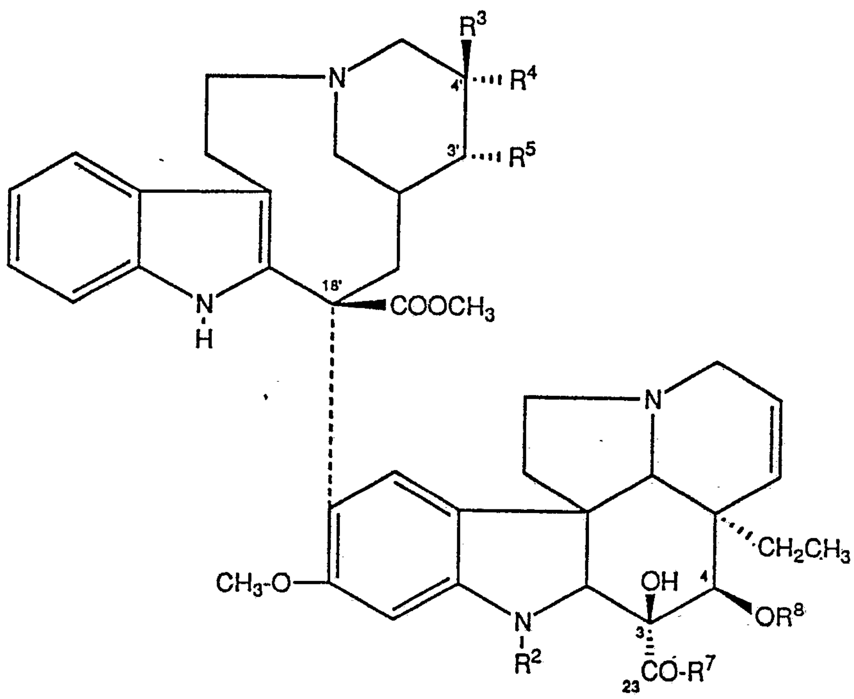

II

wherein $R^7$ is $NHNH_2$,

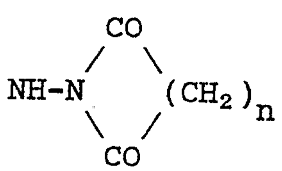

,

$O(C_{1-3}$ alkyl), $NH_2$, $NH(C_{1-3}$ alkyl), $NH$-$CH_2CH_2$-Y, 1-pyrrolidinyl or 1-piperidinyl, wherein n is 2-4 and Y is Cl, $OCH_3$ or $SCH_3$; $R^8$ is H, $(C_{1-3}$ alkyl)-CO, chloro-substituted $(C_{1-3}$ alkyl)-CO or $R^9$ wherein $R^9$ is $COXCONHNH_2$, provided that either $R^7$ is $NHNH_2$ or $R^8$ is $R^9$, but $R^7$ cannot be $NHNH_2$ when $R^8$ is $R^9$ and $R^8$ cannot be $R^9$ when R is $NHNH_2$.

The invention further provides a method of use of the conjugates of formula I as oncolytic agents, and injectable pharmaceutical formulations comprising the conjugates of formula I associated with a pharmaceutically acceptable diluent therefor.

Indole-dihydroindole alkaloids useful in preparing the hydrazides which form the conjugates of this invention, can be represented by the following 2-dimensional structure

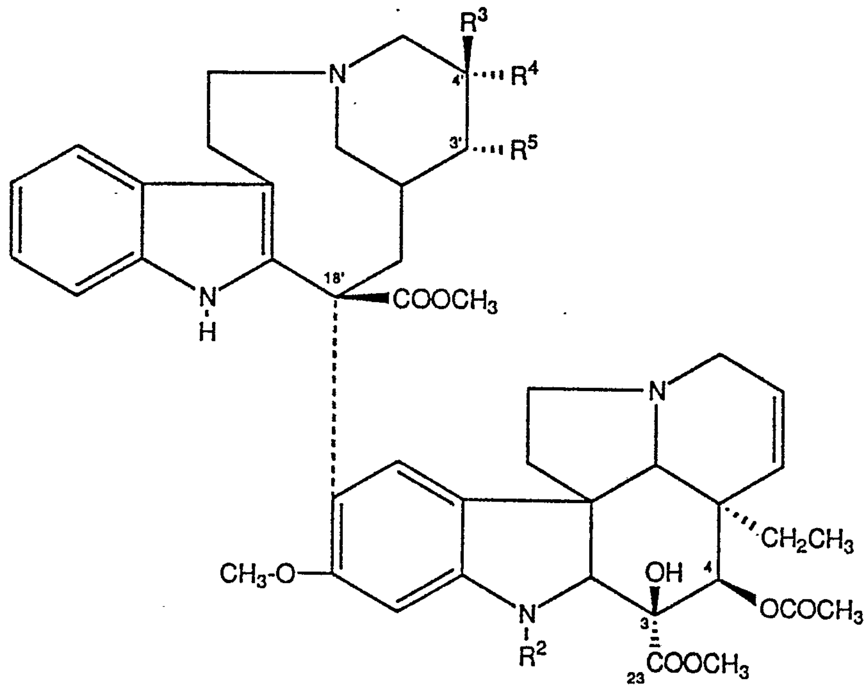

III

wherein $R^2$, $R^3$, $R^4$, and $R^5$ have their previous meanings.

In formula III above, where $R^2$ is methyl, $R^3$ is hydroxyl, $R^4$ is ethyl and $R^5$ is H, VLB (vinblastine) is represented; where $R^2$ is formyl, $R^3$ is hydroxyl, $R^4$ is ethyl and $R^5$ is H, vincristine (VCR) is represented; where $R^2$ is methyl, $R^3$ is ethyl, $R^4$ is hydroxyl, and $R^5$ is H, leurosidine is represented; where $R^2$ is methyl or formyl, $R^3$ is ethyl and $R^4$ and $R^5$ taken together with the carbons to which they are attached form an alphaepoxide ring, leurosine and leuroformine, respectively, are represented; where $R^2$ is methyl, $R^3$ is ethyl, and $R^4$ and $R^5$ are H, deoxy VLB "B" (4′-deoxyleurosidine or 4′-epideoxy VLB) is represented; where $R^2$ is methyl, $R^4$ is ethyl and $R^3$ and $R^5$ are H, deoxy VLB "A" or 4′-deoxy VLB is represented; and where $R^2$ is CHO, $R^3$ is ethyl, $R^4$ and $R^5$ are H, 4′-epideoxyvincristine (1-formyl-1-desmethyl-4′-deoxyleurosidine) is represented.

Literature references to the parent vinca alkaloids (III) are as follows: leurosine (U.S. Patent. No. 3,370,057), VLB (U.S. Patent No. 3,097,137), leurosidine (vinrosidine) and leurocristine (to be referred to hereafter as vincristine) (both U.S. Patent No. 3,205,220), desmethyl VLB (U.S. Patent No. 3,354,163), 4′-epivincristine (U.S. Patent No. 4,143,041), leuroformine, formylleurosine (U.S. Patent No. 4,279,816), and deoxy VLB "A" and "B" [Tetrahedron Letters, 783 (1958)].

The hydrazides of formula II useful in forming the conjugates of this invention are prepared differently, depending on whether the hydrazide is attached at C-3 or C-4. The C-3 hydrazides are prepared by the procedure of U.S. Patent 4,203,898, col 12, line 65 et seq. and Example 3, col. 18. In this procedure, anhydrous hydrazine is reacted with a vinca alkaloid according to formula III in ethanol in a sealed tube at about 60°C. The product of this reaction is a 4-desacetyl 3-carboxhydrazide since the acetoxy group at C-4 is hydrolysed under the basic reaction conditions. If it is desirable to prepare an ester at C-4 ($R^8$ in II is ($C_{1-3}$ alkyl)-CO or chloro ($C_{1-3}$-alkyl)-CO), the C-3 carboxhydrazide wherein $R^8$ is H is first protected by reaction with acetone to form an $N^2$-propylidene derivative. With the acylable $NH_2$ group of the hydrazide effectively protected against acylation, this "protected" derivative can then be acylated in routine fashion with an acyl halide (chloroacetyl chloride for example) or an acyl anhydride (propionic anhydride for example). The protecting group can then be removed by treatment with acid. If acylation also occurs on the C-3 hydroxyl, as it usually does, this C-3 acyl group can be preferentially removed by treatment with wet silica gel--see Hargrove, U.S. Patent 3,392,173.

When the hydrazide group is part of a C-4 chain (i.e., part of $R^9$ in 11 above), the 4-desacetyl starting materials are prepared as follows, depending on the nature of the C-3 group: where C-3 is an ester group, the 4-desacetyl derivative is prepared by the procedure of Hargrove, U.S. patent 3,392,173, Examples 1-5; where C-3 is an amide group, the 4-desacetyl is prepared by the procedure of Conrad et al., supra, or Cullinan, U.S. patent 4,203,898. This procedure involves preparation of a C-3 carboxhydrazide which is accompanied by hydrolysis at C-4. The hydrazide is then converted to the azide and the azide reacted with ammonia or a primary amine of the structure $NH_2$-($C_{1-3}$ alkyl), or with $NH_2$-$CH_2$-$CH_2$-S-$CH_3$, $NH_2$-$CH_2$-$CH_2$-O-$CH_3$, pyrrolidine, piperidine or $NH_2$-$CH_2$-$CH_2$-Cl. A preferable method of preparing this last compound, the chloroethylamide to yield the desired C-3 carboxamide C-4 hydroxyl derivative is by decomposition of a 3″-(β-chloroethyl)-3-spiro-5″-oxazolidine-2″,4″-dione of a vinca alkaloid according to formula III above. The

preparation of these oxazolidinedione derivatives is set forth in Miller and Gutowski, U.S. Patent RE 30,560. The preparation of the β-chloroethylamide from the above oxazolidinedione is set forth in Example 1 of Miller and Gutowski U.S. patent 4,357,334. (Example 2 of the same patent prepares the same β-chloroethylamide using the classical azide process). The primary amide (R in formula I is $NH_2$) can also be prepared by direct ammonolysis or by reduction of the hydrazide with Raney Nickel -- see also U.S. patent 4,203,898. Finally, when $R^7$ in II is

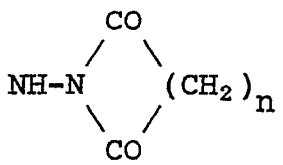

where n is 2-4; i.e., a cyclic succinimide, glutarimide, or adipimide, respectively, the 4-hydroxyl derivatives are prepared by the procedure set forth by Cullinan, U.S. Patent 4,667,030.

Certain vinca hydrazides are preferred for use in preparing the conjugates of the present invention. While all of the conjugates of Formula I are useful, those of the following classes are particularly desirable. It will be understood that the following classes may be combined to obtain further, more limited classes of preferred compounds.

1) R is NHN=;
2) $R^1$ is $R^6$;
3) R is alkoxy or alkylamino;
4) $R^1$ is hydrogen;
5) $R^2$ is methyl or CHO;
6) $R^3$ is OH or ethyl;
7) $R^4$ is hydrogen or ethyl;
8) $R^5$ is hydrogen;
9) X is alkylene;
10) X is $C_{1-4}$ straight chain alkylene.

The following list illustrates some of the preferred vinca hydrazides of Formula II which may be employed in this invention and their common names as known in the art:

4-Desacetyl-VLB-3-carboxhydrazide $R^7$ = $NHNH_2$; $R^8$ = $R^5$ = hydrogen: $R^2$ = methyl: $R^3$ = hydroxy: $R^4$ = ethyl)

4-Desacetyl-VLB-4-hemisuccinate hydrazide ($R^7$ = $OCH_3$; $R^8$ = $COCH_2CH_2CONHNH_2$; $R^2$ = methyl; $R^3$ = hydroxy; $R^4$ = ethyl; $R^5$ = hydrogen)

4-Desacetyl-VLB-3-carboxhydrazide-$N^2$-succinimide-4-hemisuccinate hydrazide

($R^7$ = 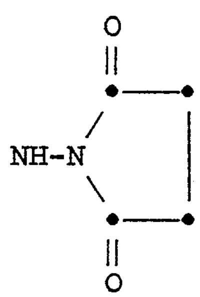 ;

$R^8$ = $COCH_2CH_2CONHNH_2$; $R^2$ = methyl; $R^3$ = hydroxy; $R^4$ = ethyl; $R^5$ = hydrogen)

4-Desacetyl-VLB-3-carboxamide-4-hemisuccinate hydrazide ($R^7$ = $NH_2$; $R^8$ = $COCH_2CH_2CONHNH_2$; $R^2$ = methyl; $R^3$ = hydroxy; $R^4$ = ethyl; $R^5$ = hydrogen)

4-Desacetyl-VLB-4-hemiglutarate hydrazide ($R^7$ = $OCH_3$; $R^8$ = $COCH_2CH_2CH_2CONHNH_2$; $R^2$ = methyl: $R^3$ = hydroxy; $R^4$ = ethyl; $R^5$ = hydrogen)

4-Desacetyl-VCR-4-hemisuccinate hydrazide $R^7$ = $OCH_3$; $R^8$ = $COCH_2CH_2CONHNH_2$; $R^2$ = CHO; $R^3$ = hydroxy; $R^4$ = ethyl; $R^5$ = hydrogen)

4-Desacetyl-4′-epideoxy-VLB-4-hemisuccinate hydrazide ($R^7$ = $OCH_3$; $R^8$ = $COCH_2CH_2CONHNH_2$; $R^2$ = methyl; $R^3$ = ethyl; $R^4$ = $R^5$ = hydrogen)

4-Desacetyl-VLB-3-carboxhydrazide-$N^2$-glutarimide-4-hemisuccinate hydrazide

($R^7$ = 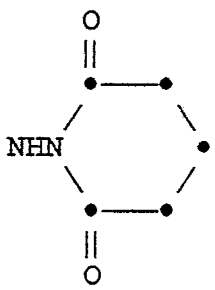 ;

$R^8$ = $COCH_2CH_2CONHNH_2$; $R^2$ = methyl; $R^3$ = hydroxy; $R^4$ = ethyl; $R^5$ = hydrogen)

4-Desacetyl-VCR-3-carboxhydrazide ($R^7$ = $NHNH_2$; $R^8$ = $R^5$ = hydrogen; $R^2$ = CHO; $R^3$ = hydroxy; $R^4$ = ethyl)

4-Desacetyl-4′-epideoxy-VLB-3-carboxhydrazide ($R^7$ = $NHNH_2$; $R^8$ = $R^4$ = $R^5$ = hydrogen; $R^2$ = methyl; $R^3$ = ethyl)

4-Desacetyl-VLB-3-ethylcarboxamide-4-hemisuccinate hydrazide ($R^7$ = $NHCH_2CH_3$; $R^8$ = $COCH_2CH_2CONHNH_2$; $R^2$ = methyl; $R^3$ = hydroxy; $R^4$ = ethyl; $R^5$ = hydrogen)

4-Desacetyl-VLB-3-methoxyethylcarboxamide-4-hemisuccinate hydrazide ($R^7$ = $NHCH_2CH_2OCH_3$; $R^8$ = $COCH_2CH_2CONHNH_2$; $R^2$ = methyl; $R^3$ = hydroxy; $R^4$ = ethyl; $R^5$ = hydrogen)

4-Desacetyl-VLB-4-propionyl-3-carboxhydrazide ($R^7$ = $NHNH_2$; $R^8$ = $COCH_2CH_3$; $R^2$ = methyl; $R^3$ = hydroxy; $R^4$ = ethyl; $R^5$ = hydrogen).

The preparation of C-4 derivatives of these dimeric indole-dihydroindole alkaloids variously substituted at C-3 (see $R^7$ in formula II) is carried out by reacting compounds according to II where $R^8$ is H and $R^7$ and $R^2$-$R^5$ have their assigned meanings in a multistep process as outlined in British application 2,137,202A or Cullinan, U.S. Patent 4,667,030.

The other component of the present conjugates is an oxidized antibody of the IgG class, preferably a monoclonal antibody (MoAb), which antibody contains from about 6% to about 18% of carbohydrate. Thus, the antibodies used in the present invention are quite unusual, because typical IgG immunoglobulins contain only about 2-5% of carbohydrate. (Authorities have estimated different figures for the carbohydrate content of IgG. Goding, Monoclonal Antibodies: Principles and Practice, Academic Press, 1983, p. 9, reports 2-3%, and Ghose et al., J. Immunol. Methods 59, 130 (1983), says 3%.)

The term "antibody" is used in this document to include Fab, Fab′ and $F(ab')_2$ fragments of antibodies of the class described in the paragraph just above.

The antibody may be one wherein the unusual amount of carbohydrate is located on the light chains. However, the heavy chains may contain the unusual amount of carbohydrate, or the Fab portion of the antibody as a whole may contain the unusual amount of carbohydrate. On the other hand, the unusual amount of carbohydrate may be contained, or partially contained, in the $F_c$ portion of the antibody.

While the antibodies used in the present invention contain from about 6% to about 18% of carbohydrate, preferred classes of antibodies contain amounts of carbohydrate in various other ranges, as follows:

1) about 6% to about 15%;
2) about 6% to about 12%;
3) about 8% to about 15%;
4) about 8% to about 12%;
5) about 6% to about 10%;
6) about 10% to about 15%.

Techniques for the production of antibodies from the serum of immunized animals or by culturing hybridomas secreting monoclonal products are well known. Hybridomas may also be modified by genetic engineering techniques to produce antibodies having defined properties, such as the unusual carbohydrate content of the presently used antibodies. Purified antibodies may also be chemically modified to increase their carbohydrate content. Representative antibodies used in the present invention include, for example, mono- or polyclonal antibodies to

(i) human or animal tumor associated antigens;
(ii) human B- and T-cell antigens;
(iii) human Ia antigens;
(iv) viral, fungal and bacterial antigens; and
(v) cells involved in human inflammatory or allergic reactions.

Of the preferred antibodies to human or animal tumor associated antigens there may be mentioned:

(i) IgG from goats or sheep immunized with carcinoembryonic antigen;
(ii) IgG from rabbit antiacute lymphoblastic leukemia serum;
(iii) IgG from various primate antisera raised against acute lymphoblastic leukemia, acute myeloblastic leukemia, chronic lymphoblastic leukemia and chronic granulocytic leukemia;
(iv) IgG from goats or sheep immunized with lung carcinoma cells, or cellular fractions;

(v) monoclonal IgG from mouse hybridomas secreting anti-human colorectal carcinoma antibodies;
(vi) monoclonal IgG from mouse hybridomas secreting anti-human melanoma antibodies;
(vii) monoclonal IgG from mouse hybridomas that secrete antibodies reacting with human leukemia cells;
(viii) monoclonal IgG from mouse hybridomas secreting antibodies reacting with human neuroblastoma cells;
(ix) monoclonal IgG from mouse hybridomas secreting antibodies reacting with human breast cancer antigens;
(x) monoclonal IgG from mouse hybridomas secreting antibodies reacting with human ovarian carcinoma cells;
(xi) monoclonal IgG from mouse hybridomas secreting antibodies reacting with human osteosarcoma cells, with human pancreatic carcinoma cells, with human prostatic carcinoma cells etc.;
(xii) monoclonal IgG from mouse hybridomas secreting antibodies to adenocarcinomas including lung, renal, breast and pancreas;
(xiii) monoclonal IgG from mouse hybridomas secreting antibodies reacting with human squamous carcinoma cells;
(xiv) monoclonal IgG from human hybridomas (hybridomas which secrete antibodies to the human tumor-associated antigen including, but not limited to, those monoclonals above)..

Preferred conjugates are those prepared from monoclonal antibodies, especially those which recognize human cancer cells such as adenocarcinoma, sguamous cell carcinoma, transitional cell carcinoma, melanoma, neuroblastoma, small cell carcinoma, leukemia, lymphoma, and sarcoma.

The antibody is prepared for reaction with the vinca hydrazide by oxidizing it with periodate or another suitable oxidizing agent so that bonds between vicinal diols in surface carbohydrates are ruptured and aldehyde groups on either side of the original bond are produced thereby. It will be understood by chemists that the aldehyde group, being in solution in the presence of other functional reagents, may actually be in the form of an acetal, hemiacetal, aminal, hemiaminal or the like.

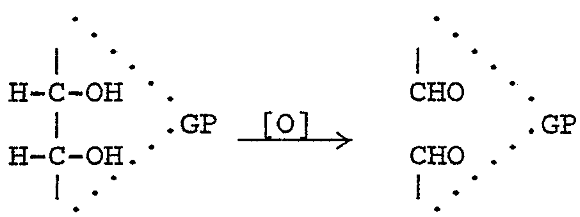


carbohydrate part structure

dialdehyde product

The proportion of the carbohydrate units which are oxidized, and the number of dialdehyde units produced, is a result of the following factors as will be appreciated by those skilled in the art: amount of periodate employed, general reaction conditions of oxidation (e.g., time, temperature, solvent, concentration, etc.), number of vicinal diol carbohydrate units present on the antibody, and their accessibility to the oxidizing reagent.

Alternatively, or in combination with the periodate oxidation above, one may employ enzymatic oxidation with galactose oxidase. This is a more selective and restrictive reagent catalyzing the conversion of the 6-$CH_2OH$ position of galactose residues on the carbohydrate units to an aldehyde functionality. This 6-$CH_2OH$ group must be unsubstituted to allow for successful oxidation, and unmasking a blocked group, such as a group wherein a sialic acid residue is attached, may be accomplished with the enzyme neuraminidase. As above, the number of aldehyde moieties so produced will be a function of many variables.

The oxidized antibody is then conjugated with a vinca hydrazide of Formula I in a nucleophilic reaction wherein the lone pair of electrons on the terminal nitrogen of the hydrazide group seeks the aldehyde group of the oxidized antibody.

In general, each mole of antibody is conjugated to a number of moles of vinca hydrazide. Indeed, one of the advantages of the present invention is the large amount of drug which can be loaded on each molecule of antibody. The term "conjugation ratio" is used for the average number of moles of vinca hydrazide per mole of antibody. Conjugation ratios from about 5 to about 35 are usual in the present invention, and conjugation ratios in the range of from about 5 to about 25 are preferred. Further preferred ranges of conjugation ratios are from about 10 to about 20, from about 15 to about 25, and from about 5 to about 15.

The conjugation of the vinca hydrazides with oxidized antibodies is accomplished by standard methods known in the art. In general, a solution of the vinca of formula II in a water-miscible solvent such as dimethylformamide is added to a chilled buffered aqueous solution of the oxidized antibody. The vinca may

also be used as the sulfate salt, in which case no organic solvent is needed. Temperatures of about 0-8° C are preferred and a 0.1N sodium acetate buffer is normally employed. The reaction is best carried out in the dark and under an inert atmosphere. The reaction is usually complete in about 10-24 hours and the resulting conjugate may be purified by standard methods, such as by chromatography over Sephadex.

The MoAb used in the following Examples is ZCE-025, a murine antibody of the IgG: subclass which reacts with carcinoembryonic antigen. It is under development by Hybritech Incorporated, Abdel-Nabi et al., Radiology 164, 617-21 (1987). The antibody has been studied for some time under the name Monoclonal Antibody 35, by J.-P. Mach and associates, particularly as a diagnostic agent for cancer. Delaloye et al., J. Clin. Invest. 77, 301-11 (1986); Haskell et al.., . Cancer Res. 43, 3857-64 (1983); Sutherland et al., Cancer Res. 47, 1627-33 (1987).

ZCE-025 has been found to have light chains of unusually high molecular weight, 28,000, by analysis on SDS-PAGE under reducing conditions. The method was that described by Laemmli, U.K. Nature (London) 227, 680-85 (1970). Treatment of ZCE-025 with Endoglycosidase F (Elder et al., Proc. Nat. Acad. Sci. USA 70, 4540-44 (1982) caused much of the light chain to migrate on SDS-PAGE in the same region as the control, indicating that the high weight of the light chain is caused by carbohydrate.

## Example 1

A solution was prepared by dissolving 10 mg of ZCE-025 in 1 ml of a 0.1M sodium acetate buffer at pH 5.6 (29.3 g sodium acetate, 2.44 ml acetic acid plus sufficient sterilized water to make 4 L of buffer). To the solution at 0-2° C was added 2.1 mg of sodium metaperiodate with rapid stirring.

The mixture was stirred for 10 minutes at about 0-2° C in the dark and was then quenched by the addition of 0.004 ml of a 12.5M solution of ethylene glycol in sterile water. The new mixture was stirred at 0-2° C for 5 minutes in the dark and was then centrifuged to leave a clear supernatant and a white pellet. The supernatant was loaded onto a Sephadex G25 (medium mesh) gel column and the product eluted with the same sodium acetate buffer. The eluate was monitored with UV light at 280 nm. Concentration of the oxidized product was assessed in each eluate fraction at 280 nm.

The above eluate solution of oxidized product had a final protein concentration of 2.5 mg/ml and total volume of 3.1 ml. The solution was cooled to about 4° C.

A 13.4 mg portion of 4-desacetyl VLB 3-carboxhydrazide sulfate was added to the chilled buffered MoAb solution. The reaction vessel was flushed with nitrogen gas and then sealed. The reaction mixture was stirred in the cold and dark with magnetic stirring for 16 hours. The reaction vessel was then unsealed and the clear, pale yellow reaction mixture was chromatographed over Sephadex G25 gel preequilibrated with pH = 7.4 phosphate buffered saline (0.01M phosphate, 0.15M NaCl) which was also used as the eluant. The conjugate was eluted first followed by unreacted 4-desacetyl VLB 3-carboxhydrazide. The yield of conjugate obtained was 5.7 mg in 4.8 ml. The conjugation ratio was about 5.2 moles of 4-desacetyl-VLB-3-carboxhydrazide per mole of MoAb, as determined by dual-wavelength UV spectroscopy at 280 and 270 nm.

## Example 2

A 10 mg sample of the same MoAb in 1 ml of acetate buffer was oxidized for 21 minutes at 0° C with 34 mg of sodium metaperiodate. The reaction was then quenched with 0.064 ml of 12.5M ethylene glycol, and the product was purified as in Example 1 to obtain 9.7 mg of oxidized MoAb in 4 ml of solution. To that solution at 0° C was added 17 mg of 4-desacetyl-VLB-3-carboxhydrazide sulfate, and the mixture was left at 4° C in the dark for 16 hours. The reaction mixture was then centrifuged for 20 minutes, and the supernatant was worked up as described in Example 1 to obtain 0.6 mg of product having a conjugation ratio of 27.2.

## Example 3

A process similar to that of Example 1 was carried out. The amount of sodium metaperiodate was 2.1 mg, and the oxidation went on for 21 minutes. An 8.8 mg yield of oxidized MoAb was obtained, and it was reacted with 19 mg of the vinca hydrazide and worked up as described in Example 2 to obtain 6.9 mg of conjugate with a conjugation ratio of 8.6.

## Example 4

The process of Example 3 was repeated, except that the amount of sodium metaperiodate was 8.6 mg and the amount of ethylene glycol was 0.016 ml. The yield of oxidized MoAb was 8.6 mg, which was reacted with 18 mg of the vinca hydrazide. Five mg of the desired conjugate, having a conjugation ratio of 13.8, was obtained.

Example 5

The process of Example 3 was repeated again, except that the amount of sodium metaperiodate was 8.6 mg and the amount of ethylene glycol was 0.016 ml, and the oxidation time was 10 minutes. A yield of 8.1 mg of oxidized MoAb was obtained. Reaction with 19 mg of the vinca hydrazide produced 6.5 mg of conjugate having a conjugation ratio of 10.3.

The conjugate prepared in the examples above were determined by radioimmunoassay and immunofluorescence to bind to antigen-positive LS174T human colon adenocarcinoma cells, at least as well as does unconjugated ZCE-025. Analysis by immunofluorescence showed that the conjugates did not bind to antigen-negative M14 human melanoma cells.

Example 6

A 374 mg portion of antibody ZCE-025 was dialyzed into 28.8 ml of sodium acetate buffer at pH 5.6, 8.1 ml of additional buffer was added, and then it was combined with 0.53 ml of aqueous sodium periodate solution, containing 150 mg/ml. The mixture was stirred for 10 minutes in the dark, and then the reaction was quenched by addition of ethylene glycol. The reaction mixture was then centrifuged, and the supernatant was chromatographed over Sephadex G25, eluting with sodium acetate buffer. The antibody-containing fractions were combined and analyzed by UV spectroscopy at 280 nm, which showed that 355 mg of oxidized antibody had been recovered. It was diluted to a concentration of 2.8 mg/ml in sodium acetate buffer, and to it was added 554 mg of 4-desacetyl-VLB-3-carboxhydrazide. The reaction mixture was allowed to stand overnight at 4°C, and the mixture was then purified by chromatography on Sephadex G25, eluting with physiological buffered saline at pH 7.4. The product-containing fractions were combined, and were filtered through 0.2 $\mu$ membrane. A total of 304 mg of conjugate was recovered, at a concentration of 13.5 mg/ml, with a conjugation ratio of 5.0 moles of drug per mole of antibody.

Example 7

The process of Example 6 was substantially repeated, with modifications to prepare a product of higher conjugation ratio. The amount of sodium periodate solution was 6.4 ml at 150 mg/ml concentration, and the oxidation reaction was allowed to proceed for 21 minutes. The same amount of vinca drug was supplied, and the product amounted to 187 mg of conjugate, at a concentration of 8.9 mg/ml, with a conjugation ratio of 9.7 moles of drug per mole of protein.

The conjugates of Examples 6 and 7 were tested against xenografts of the LS174T tumor growing in female Charles River nude mice. LS174T is a human, colorectal tumor which produces carcinoembryonic antigen. The test was begun by implanting each mouse subcutaneously with $10^7$ cells of the tumor. On each of days 7, 10, 14 and 17 after implantation, each mouse was injected with a dose of the conjugate which provided 3 mg/kg body weight of vinca drug. The size of the tumors induced by implantation was measured at various times, up to 42 days after implantation. Each treatment group consisted of five mice, except for the untreated control group of ten mice. A group which received unconjugated 4-desacetyl-VLB-3-carboxhydrazide was also included in the test.

After 42 days, the control mice had very large tumors averaging about 23.5 g. All of the mice which received the conjugate of Example 6 had died. One animal receiving the conjugate of Example 7 had died, and the other four had tumors averaging about 4.5 g. Three of the mice receiving unconjugated drug had died, and the other two had tumors also averaging about 4.5 g.

In another test, carried out in the same manner, the tumors grew much more slowly. At 34 days after implantation, the control mice had tumors weighing about 3.7 g. All of the mice receiving unconjugated drug had died. The mice receiving the conjugates of Examples 6 and 7 both had tumors averaging only about 0.1 g; however, four of the mice receiving the conjugate of Example 6 had died, and none of those receiving Example 7 had died.

In a similar test, the conjugates were administered at rates giving only 2 mg/kg of vinca drug. Here, the control mice had tumors averaging about 23.5 g, as in the first test. None of the mice receiving free vinca drug or the conjugate of Example 7 had died at the 42-day measurement point; only one of the mice receiving the conjugate of Example 6 had died. The tumors of the animals receiving free vinca drug averaged about 8 g at 42 days, and the tumors of the mice receiving Examples 6 and 7 both averaged about 5 g.

It has been found that the conjugates of this invention are quite safe, and that, most unexpectedly, conjugates having high conjugation ratios are even more safe than those of lower vinca drug content. Accordingly, the conjugates provide a most advantageous way to administer relatively large doses of the antineoplastic vinca drugs with unexpected safety to the patient.

The novel conjugates of the invention are useful in the treatment of cancers and as such are preferably prepared for use in formulations suitable for injection. Thus the invention includes a pharmaceutical formulation, for example an injectable preparation comprising a conjugate of the invention together with a

pharmaceutically-acceptable carrier or diluent such as are well known in the art. The formulation is preferably in unit dosage form, each dosage containing, for example, from 0.01 to 10 mg of the active ingredient (in terms of the vinca drug moiety).

The novel conjugates are effective over a wide dosage range and dosages per week, for example, for the treatment of adult humans suffering from cancer will normally fall within the range of 0.01 to 10 mg/kg (vinca drug moiety), more usually in the range of from 0.03 to 9 mg/kg. However it will be understood that the amount of conjugate actually administered will be determined by a physician in the light of the relevant circumstances, including the condition to be treated and the chosen route of administration.

**Claims**

1. A conjugate of the formula

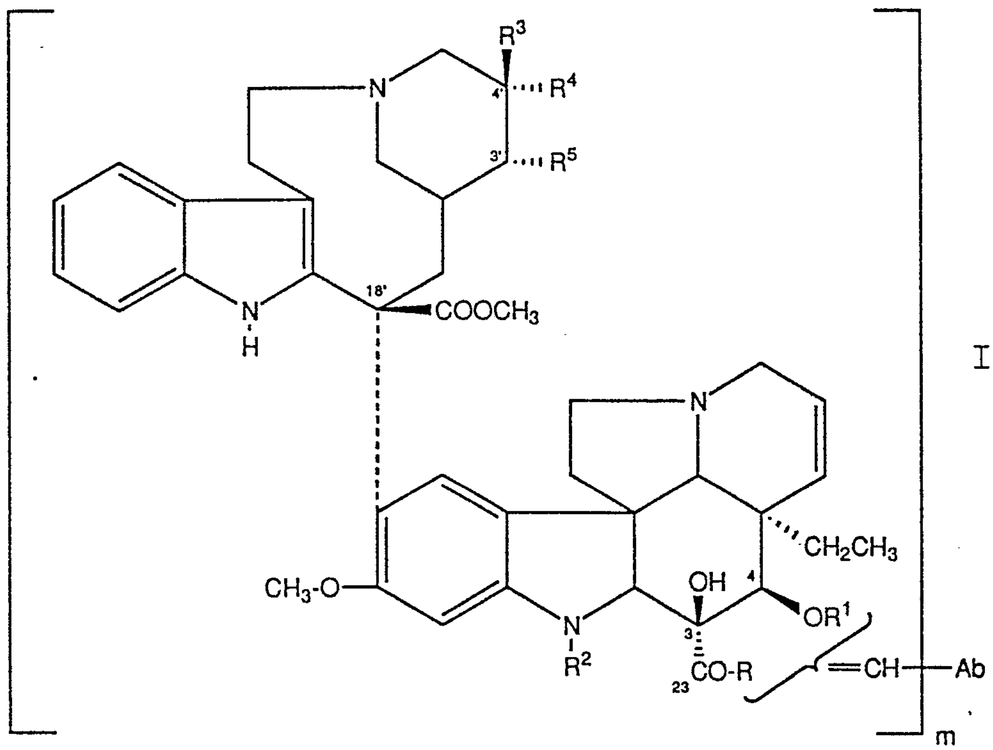

I

wherein $R^2$ is H, $CH_3$ or CHO; when $R^4$ and $R^5$ are taken singly, $R^5$ is H, and one of $R^3$ and $R^4$ is ethyl and the other is H or OH; when $R^4$ and $R^5$ are taken together with the carbons to which they are attached, they form an oxirane ring in which case $R^3$ is ethyl; R is NHN=,

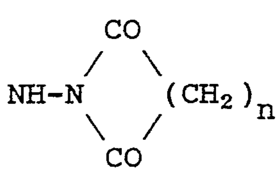

,

$O(C_{1-3}$ alkyl), $NH_2$, $NH(C_{1-3}$ alkyl), $NH-CH_2CH_2-Y$, 1-pyrrolidinyl or 1-piperidinyl, wherein n is 2-4 and Y is Cl, $OCH_3$ or $SCH_3$; $R^1$ is H, $(C_{1-3}$ alkyl)-CO, chloro-substituted $(C_{1-3}$ alkyl)-CO or $R^6$ wherein $R^6$ is COXCONHN= wherein X is $C_{1-4}$ straight chain alkylene, $C_{2-8}$ branched chain alkylene, $C_{2-4}$ alkenylene, $C_{3-4}$ alkynylene, $C_{3-6}$ cycloalkylene, phenylene, hydroxysubstituted $C_{1-4}$ alkylene, or a direct bond, provided that either R is NHN= or $R^1$ is $R^6$, but R cannot be NHN= when $R^1$ is $R^6$ and $R^1$ cannot be $R^6$ when R is NHN= ; m is an integer from about 5 to about 35; Ab is an antibody of the IgG class, containing from about 6% to about 18% of carbohydrate.

2. A conjugate of claim 1 wherein the antibody is a monoclonal antibody.

3. A conjugate of claim 1 or 2 wherein the antibody is adapted for recognition of antigens on the surface of unwanted mammalian cells.

4. A conjugate of claim 1, 2 or 3 wherein the antibody is adapted for recognition of human cancer cells selected from the group consisting of adenocarcinoma, squamous cell carcinoma, transitional cell carcinoma, melanoma, neuroblastoma, small cell carcinoma, leukemia, lymphoma, and sarcoma.

5. A conjugate of any one of claims 1 to 4 wherein R is NHN= ; $R^1$ and $R^5$ are hydrogen; $R^2$ is methyl; $R^3$ is hydroxy; and $R^4$ is ethyl.

6. A conjugate of any one of claims 1 to 4 wherein R is $OCH_3$; $R^1$ is $COCH_2CH_2CONHN$= ; $R^2$ is methyl; $R^3$ is hydroxy; $R^4$ is ethyl; and $R^5$ is hydrogen.

7. A conjugate of any one of claims 1 to 4 wherein R is $NH_2$; $R^1$ is $COCH_2CH_2CONHN$= ; $R^2$ is methyl; $R^3$ is hydroxy; $R^4$ is ethyl; and $R^5$ is hydrogen.

8. A conjugate of any one of the preceding claims for use in the treatment of cancer.

9. A pharmaceutical formulation comprising as an active agent a conjugate as claimed in any one of claims 1 to 7 associated with one or more pharmaceutically acceptable carriers or diluents therefor.

10. A process for preparing a conjugate as claimed in any one of claims 1 to 7 comprising reacting an oxidized antibody of the IgG class containing from about 6% to about 18% of carbohydrate, with a vinca hydrazide of the formula

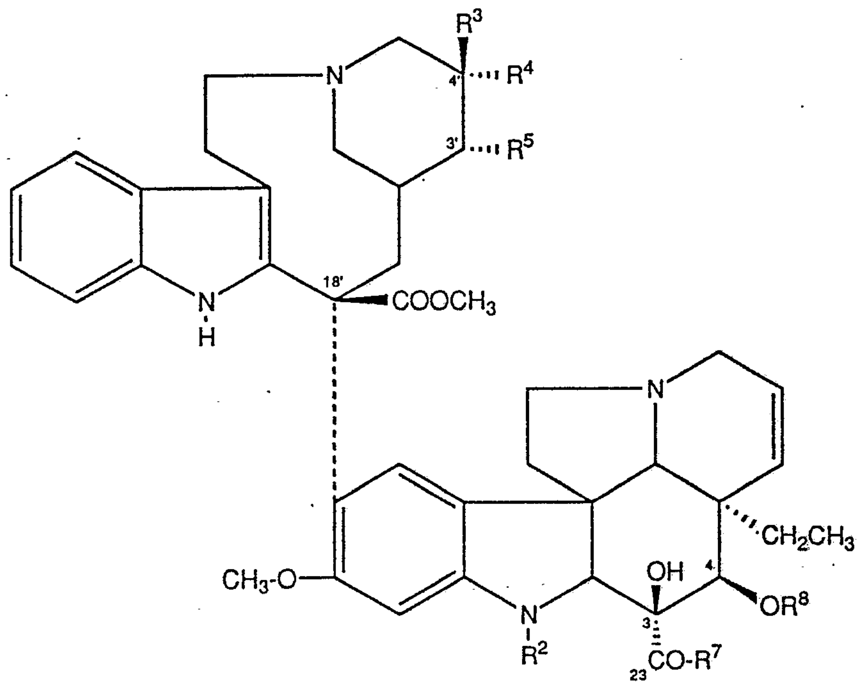

II

wherein $R^7$ is $NHNH_2$,

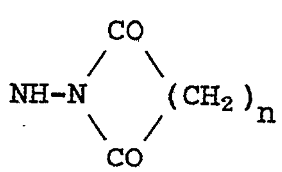

,

O($C_{1-3}$ alkyl), $NH_2$, NH($C_{1-3}$ alkyl), NH-$CH_2CH_2$-Y, 1-pyrrolidinyl or 1-piperidinyl, wherein n is 2-4 and Y is Cl, $OCH_3$ or $SCH_3$; $R^8$ is H, $C_{1-3}$ alkyl)-CO, chloro-substituted ($C_{1-3}$ alkyl)-CO or $R^8$ wherein $R^9$ is $COXCONHNH_2$, provided that either $R^7$ is $NHNH_2$ or $R^8$ is $R^9$, but $R^7$ cannot be $NHNH_2$ when $R^8$ is $R^9$ and $R^8$ cannot be $R^9$ when R is $NHNH_2$.

**Claims for the Following Contracting State: ES + GR.**

1. A process for preparing a conjugate of the formula

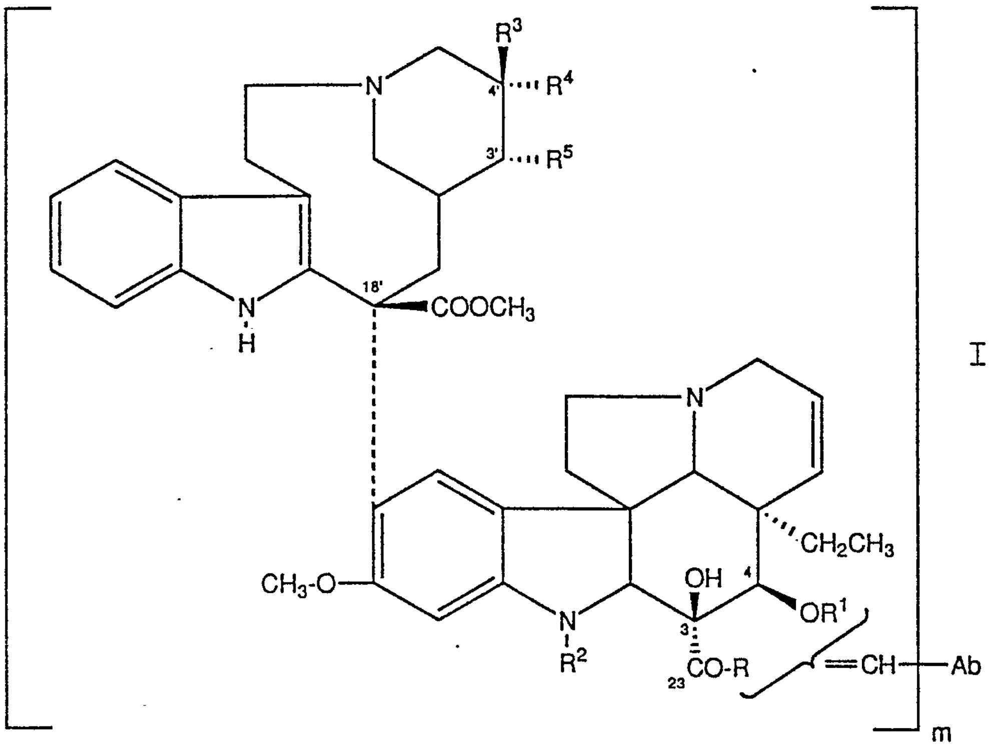

I

wherein $R^2$ is H, $CH_3$ or CHO; when $R^4$ and $R^5$ are taken singly, $R^5$ is H, and one of $R^3$ and $R^4$ is ethyl and the other is H or OH; when $R^4$ and $R^5$ are taken together with the carbons to which they are attached, they form an oxirane ring in which case $R^3$ is ethyl; R is NHN=,

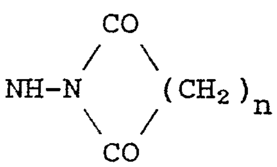

,

O($C_{1-3}$ alkyl), $NH_2$, NH($C_{1-3}$ alkyl), NH-$CH_2CH_2$-Y, 1-pyrrolidinyl or 1-piperidinyl, wherein n is 2-4 and Y is Cl, $OCH_3$ or $SCH_3$; $R^1$ is H, ($C_{1-3}$ alkyl)-CO, chloro-substituted ($C_{1-3}$ alkyl)-CO or $R^6$ wherein $R^6$ is COXCONHN= wherein X is $C_{1-4}$ straight chain alkylene, $C_{2-8}$ branched chain alkylene, $C_{2-4}$ alkenylene, $C_{3-4}$ alkynylene, $C_{3-6}$ cycloalkylene, phenylene, hydroxysubstituted $C_{1-4}$ alkylene, or a direct bond, provided that either R is NHN= or $R^1$ is $R^6$, but R cannot be NHN= when $R^1$ is $R^6$ and $R^1$ cannot be $R^6$ when R is NHN=; m is an integer from about 5 to about 35; Ab is an antibody of the IgG class, containing from about 6% to about 18% of carbohydrate; comprising reacting an oxidized antibody of the IgG class containing from about 6% to about 18% of carbohydrate, with a vinca hydrazide of the formula

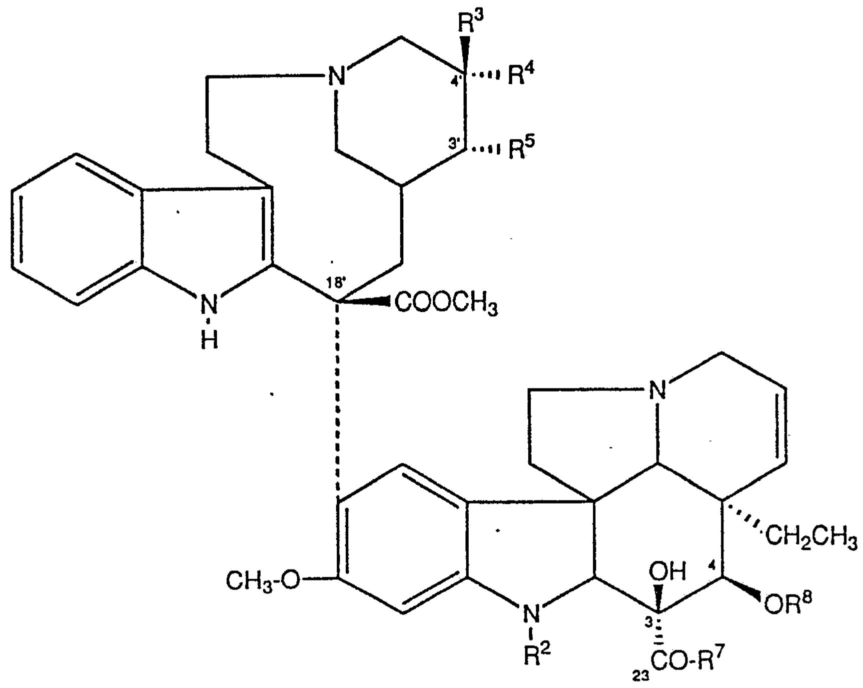

II

wherein $R^7$ is $NHNH_2$,

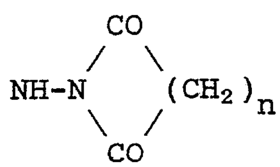

,

$O(C_{1-3}$ alkyl), $NH_2$, $NH(C_{1-3}$ alkyl), $NH-CH_2CH_2-Y$, 1-pyrrolidinyl or 1-piperidinyl, wherein n is 2-4 and Y is Cl, $OCH_3$ or $SCH_3$; $R^8$ is H, $(C_{1-3}$ alkyl)-CO, chloro-substituted $(C_{1-3}$ alkyl)-CO or $R^9$ wherein $R^9$ is $COXCONHNH_2$, provided that either $R^7$ is $NHNH_2$, or $R^8$ is $R^9$, but $R^7$ cannot be $NHNH_2$ when $R^8$ is $R^9$ and $R^8$ cannot be $R^9$ when R is $NHNH_2$.

2. A process of Claim 1 wherein the process is carried out in chilled aqueous buffered solution at a temperature of from 0° to 8° C.

3. A process of claim 1 or 2 wherein the antibody is a monoclonal antibody.

4. A process of claim 3 wherein the antibody is adapted for recognition of antigens on the surface of unwanted mammalian cells.

5. A process of claim 4 wherein the antibody is adapted for recognition of human cancer cells selected from the group consisting of adenocarcinoma, squamous cell carcinoma, transitional cell carcinoma, melanoma, neuroblastoma, small cell carcinoma, leukemia, lymphoma, and sarcoma.

6. A process for preparing an injectable pharmaceutical formulation which comprises admixing a conjugate of formula I as defined in any one of claims 1, 3, 4 and 5 with one or more pharmaceutically acceptable carriers or diluents therefor.

European Patent Office

# EUROPEAN SEARCH REPORT

Application Number

EP 89 30 0641

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 247 792 (ELI LILLY)<br>* Claims 1-14 *<br>--- | 1-10 | A 61 K 47/00<br>A 61 K 39/395 |
| P,X | FR-A-2 610 198 (IRE-CELLTARG)<br>* Whole document, in particular claims 1-18 *<br>--- | 1-10 | |
| A | EP-A-0 206 667 (ELI LILLY)<br>* Claims 9-11; page 31, lines 23-31; page 32, lines 13-16 *<br>--- | 1-10 | |
| A | EP-A-0 121 388 (LILLY INDUSTRIES)<br>* Claims 1-10; page 7, lines 18-21 *<br>----- | 1-10 | |
| | | | **TECHNICAL FIELDS SEARCHED (Int. Cl.4)** |
| | | | A 61 K |

The present search report has been drawn up for all claims

| Place of search | Date of completion of the search | Examiner |
|---|---|---|
| THE HAGUE | 18-04-1989 | SCARPONI U. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)